# EUROPEAN PATENT APPLICATION

(11) **EP 3 290 059 A1**
(43) Date of publication of application: **07.03.2018**
(21) Application number: 16462005.6
(22) Date of filing: 31.08.2016
(51) Int. Cl.: A61L 15/58

(54) **MULTILAYER MAT**

(30) Priority: 30.08.2016 HU 1600505
(71) Applicant: Semmelweis Egyetem, 1085 Budapest (HU)
(72) Inventor: Sebe, István, H-8200 Veszprém (HU); Ostorházi, Eszter, H-2049 Diósd (HU); Zelkó, Romána, H-1015 Budapest (HU)
(74) Representative: Harangozo, Gabor

(57) **Abstract**

A multilayer mat for dispensing an active agent through a body surface is disclosed. The mat comprises at least one membrane layer (20) and at least one carrier layer (21) containing the active agent. The at least one carrier layer (21) is formed by a solid nanofibrous layer made of at least one biocompatible, water-soluble polymer, said carrier layer containing the active agent in a solid state. The at least one membrane layer (20) is formed by a solid nanofibrous layer made of at least one biocompatible, water-insoluble polymer, said membrane layer being permeable for both moisture and a solution of the active agent. Each of the at least one carrier layer (21) and the at least one membrane layer (20) has a predefined thickness.

## Description

The present invention relates to a multilayer mat for dispensing an active agent through a body surface.

The cosmetic and medical use of mats containing a dermal absorption active agent is becoming more and more popular, because it allows the local use of the active agent carried by the mat. The local use allows direct dosing, through which the applied dose level can be reduced and systemic loading caused by the active agent can be minimized too.

The medical mats applied on the body skin or on an injured body surface for providing controlled drug release, having an antimicrobial active agent, allow the local use of recently underutilized drugs (e.g. colistin), which have serious side effects and, when taken orally, different level of toxicity (e.g. nephro- and neurotoxicity) can be expected. However, the use of said drugs alone or in combination with other active agents may be effective, thereby also decreasing the development of resistance against antibiotics, moreover the scale of side effects on the whole body can be reduced as well. In this regard, compositions with therapeutic indication may be considered, wherein the systemic release of the drug can presumably be replaced with a local/topical treatment.

Currently, there exists a plurality of technological solutions, in which the active agent is applied locally and through the body skin by means of a mat secured on the body skin (such as the Flector™ pain relieving patch of IBSA Pharma). These products are typically formed as multilayer mats consisting of a supporting layer and a carrier layer containing the active agent, wherein the carrier layer contacts the body skin and the active agent get into the epidermis therefrom by diffusion. In these mats the carrier layer necessarily contains the active agent in the form of a liquid solution, which is typically applied on the supporting layer as a gel or hydrogel. Before the application of the mat, a protective film is used to protect the carrier layer. One of the drawbacks of these mats is that the rate of dissolution/release of the active agent from the carrier layer directly contacting the skin surface can be controlled in a rather limited way.

In the study Fathi-Azarbayjani et al. "single and multi-layered nanofibers for rapid and controlled drug delivery" (Chem. Pharm. Bull. 58, 2010, pp. 143-146), the operation of multilayer nanofibrous mats made of different hydrophilic and hydrophobic polymers is investigated. Due to the solvent (e.g. water), the active agent immediately dissolves from the layer containing the combination of the hydrophilic polymer and the active agent, thus the release of the active agent from this layer cannot be delayed. In case of the layer containing the combination of the hydrophilic polymer and the active agent, the release rate of the active agent is determined by the degradation rate of the polymer, i.e. the release rate of the active agent cannot be controlled in a flexible way.

It is an object of the present invention to provide a mat that allows the individual adjustment of the release profile of prolonged release agents by means of the use of multiple layers of different functions.

The above objects are achieved by providing a multilayer mat for dispensing an active agent through a body surface. The mat comprising at least one membrane layer and at least one carrier layer containing the active agent. The at least one carrier layer is formed by a solid nanofibrous layer made of at least one biocompatible, water-soluble polymer, said carrier layer containing the active agent in a solid state. The at least one membrane layer is formed by a solid nanofibrous layer made of at least one biocompatible, water-insoluble polymer, said membrane layer being permeable for both moisture and a solution of the active agent. Each of the at least one carrier layer and the at least one membrane layer has a predefined thickness.

The multilayer mat may comprise a plurality of supporting layers and a plurality of carrier layers that are arranged alternately. However, the multilayer mat may comprise a single carrier layer and a single membrane layer, too. In this latter case, the thickness of membrane layer is defined as a function of the desired release rate of the active agent.

Preferably, the thickness of the membrane layer is about 0.05-1 mm, more preferably about 0.05-0.5 mm.

The multilayer mat may comprise an additional microfibrous membrane layer on the outer side of a terminal membrane layer of the multilayer mat.

The multilayer mat may comprise a fastening member for securing it to the body skin. The fastening member may be formed by a covering layer arranged on the outer side of a terminal nanofibrous or microfibrous membrane layer, said covering layer extending beyond the contour of an assembly of the at least one membrane layer and the at least one carrier layer, wherein an adhesive is provided on the free surface of the covering layer facing toward said underlying layers. Alternatively, the fastening member may be formed by an adhesive layer applied on the outer side of a terminal nanofibrous or microfibrous membrane layer, along the periphery thereof.

The membrane layer and/or the carrier layer are preferably made of a polymer which is degradable under physiological conditions, in particular a polymer which can be degraded in an enzymatic or other biological way.

The invention will now be described with reference to the drawings, in which:
Figures 1a and 1b are schematic lateral sectional and perspective views, respectively, of a multilayer mat according to the present invention.
Figures 1c illustrates, in schematic lateral sectional view, the use of the mat shown in Figures 1a-b on an injured body surface producing humour.
Figure 2 schematically illustrates the operation of the multilayer mat according to the present invention.
Figures 3a-c are lateral sectional and perspective views, respectively, of a preferred embodiment of the multilayer mat according to the present invention, and a schematic lateral sectional view of the mat showing the position of this mat on a body surface.
Figures 4a and 4b are schematic lateral sectional views of two further preferred embodiments of the multilayer mat according to the present invention.
Figures 5a-d are schematic lateral sectional views of further preferred embodiments of the multilayer mat according to the present invention.
Figure 6 shows time diagrams of the absorption rate of a prolonged release agent through an injured body surface producing humour for different embodiments of the multilayer mat according to the present invention.
Figures 7a and 7b are electron microscope images of the different nanofibrous layers of the multilayer mat according to the present invention.

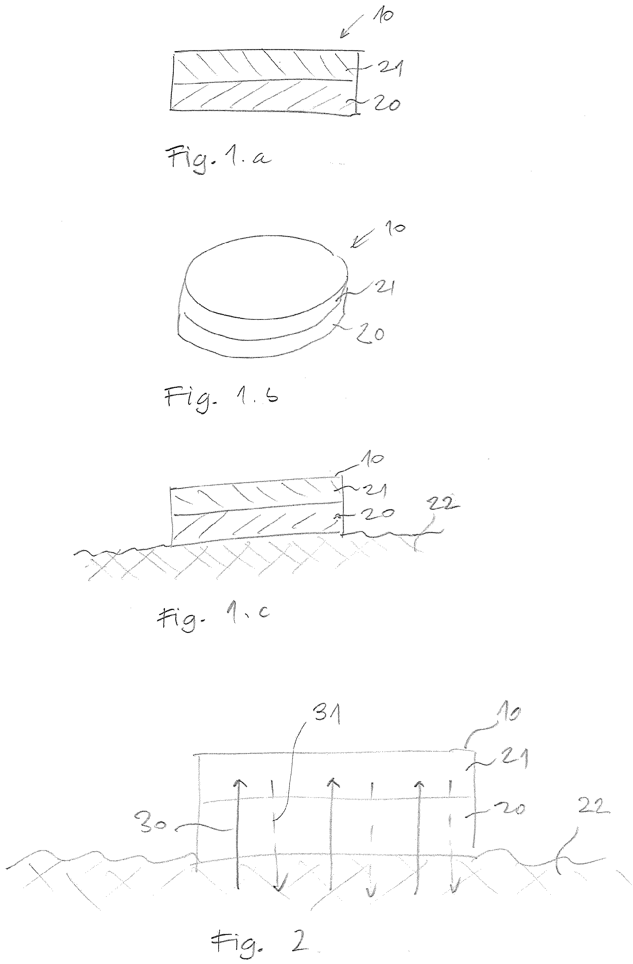

The structure of a multilayer mat 10 according to the present invention is illustrated in Figures 1 a and 1b. Figure 1a shows a schematic lateral sectional view of the multilayer mat 10 and Figure 1b depicts a schematic perspective view of the multilayer mat 10.

In the simplest case the multilayer mat 10 comprises a single membrane layer 20 and a carrier layer 21 attached thereto. The membrane layer 20 generally functions as a sort of supporting layer too. When used, the multilayer mat 10 should be placed on a moist body surface 22 and in this case the membrane layer 20 gets in contact with the body surface 22, whereas the carrier layer 21 containing the active agent does not directly contact with the body surface 22 to be treated. This arrangement is shown in Figure 1c that illustrates a schematic lateral sectional view of the layers of the multilayer mat 10 and the body surface 22. The moist body surface 22 may be, for instance, an injured body surface producing humour, wherein the humour provides the moisture for releasing the active agent, whereas for example, in a cosmetic use the moist body surface 22 may be a pre-moisturized healthy body skin. It is an important feature of the present invention that the moisture required for the release of the active agent should be provided for the mat from an external source.

The carrier layer 21 and membrane layer 20 of the multilayer mat are made of a biocompatible polymer, in particular polymer intermediates selected from a dispensatory (i.e. polymers authorized for medical use) that have physicochemical parameters which allow the generation of microscopic fibers, in particular nanofibres and microfibres. These biocompatible polymers may include, for example, the following polymers: poly(vinyl alcohol) PVA, poly(vinylpyrrolidone) (PVP), polyethylene glycol (PEG), polylactic acid (PLA), PLA-ethylene-vinyl-acetate copolymer (PEVA), polyacrylonitrile (PAN), agar, carboxymethylcellulose, polyethylene oxide (PEO), polycaprolactone (PCL), poly(acrylic acid) (PAA), chitosan, collagen, glycosaminoglycan, synthetic polixiloxan, dextran, poly(styrene-sulphonic acid-maleic acid) copolymer (PSSA-MA), poly(vinyl acetate) (PVAc), but other polymers having suitable physicochemical parameters may also be used as the material of a carrier layer and a membrane layer of the multilayer mat. It is particularly preferred that the membrane layer 20 is made of a polymer that is degradable under physiological conditions, but other known biologically degradable polymers may also be used for this purpose.

An essential feature of the multilayer mat 10 according to the present invention is that the carrier layer 21 is formed by a solid nanofibrous layer made of a water-soluble polymer or a plurality of such polymers, which renders the mat water-soluble. The carrier layer 21 comprises the active agent in solid phase. However, the membrane layer 20, which is also in the forma solid nanofibrous layer, is made of a polymer (or a combination of polymers) which is (are) not water-soluble. It is also necessary that the membrane layer 20 is permeable for both the moisture itself and the water solution of the active agent contained in the carrier layer 21.

The thickness of each layer is preferably not more than 1 mm, more preferably it ranges between 0,05 mm and 0,5 mm. With these layer thicknesses, each layer (i.e. membrane layer, carrier layer) is capable of providing its function to the required extent while keeping its resistance against mechanical stresses.

The active agent contained in the carrier layer may be any solid agent, which is dissoluble (in water) even to the least extent.

The operation of the multilayer mat 10 according to the present invention is schematically illustrated in Figure 2, which shows that the membrane layer 20 contacting directly with a moist body surface 22 (for example, with an injured body surface producing humour) sucks up the moisture 30 from the body surface 22 via the nanofibrous structure and transfers it to the carrier layer 21. The water-soluble material of the carrier layer 21 dissolves in the moisture 30 and forms a hydrogel. As a result, an aqueous solution 31 is also formed from the solid material stored in the carrier layer 21. The solution 31 containing the active agent starts to diffuse towards the membrane layer 20 due to of the difference of concentration, and through the membrane layer 20 it reaches the body surface 22 to be treated, and then it locally produces its effect through absorption in the skin. Consequently, the release of the active agent from the carrier layer 21 is diffusion-controlled.

The moisture, as a solvent, flows through the membrane layer 20 in a first direction, while the solution containing the active agent flows in the opposite direction. In the presence of moisture and under physiological conditions the membrane layer 20 can keep its fibrous structure completely or at least for a substantially long period. The membrane layer 20 can be made from non-degradable and/or biological degradable polymers. In case of biological degradable polymers, it may not be necessary to change the multilayer mat 10 during the treatment, because it will be degraded or it will be degraded and absorbed in course of time.

In the multilayer mat 10 according to the present invention, the water-soluble polymer material layer and water-insoluble polymer material layer are separated from each other, thereby it is achieved that one type of the layers (i.e. the membrane layer) functions as a membrane influencing the release rate of the active agent, whereas the other type of the layers (i.e. the carrier layer) is used to store a stable active agent in the solid phase for a long time.

The carrier layer of the multilayer mat 10 according to the present invention may comprise the active agent only in the solid phase since the water-insoluble carrier layer's polymer nanofibrous matrix dissolves and tends to form a gel in the presence of moisture. This also means that the multilayer mat 10 according to the present invention forms a system which is activated in response to moisture as an external physical action. For producing a molecular biological effect necessary to the treatment, a dissolved state of the active agent is needed, which is generated *in situ* at the beginning of the treatment. The moisture serving as a solvent is contained in the moisture (e.g. humour, sweat, etc.) produced the body surface (e.g. a wound). The active agent forms micro- and/or nano-scale solid phases within the nanofibre, generally in a fine distribution. These phases can be amorphous or crystalline, and ay combinations thereof. When the active agent is dispersed into molecules, the nanofibre containing the active agent can be considered as a solid solution, said nanofibre spontaneously transforming into a hydrogel during the absorption of the humour, which comprises the active agent in a dissolved form (as an aqueous solution).

The method of the production and the manufacturing technologies of the nanofibrous layers, used as a material of the multilayer mat 10 according to the present invention, from water-soluble and water-insoluble polymers are, are well known in the art, therefore their detailed description is omitted here. Securing the nanofibrous layers to each other and optionally, securing an outer microfibrous layer to an adjacent nanofibrous layer may be carried out in several ways.

For example, one option may be that the layers are placed on a sufficiently air-permeable plastic covering film which serves as a carrier with an adhesive strip along its periphery. The adhesive strip has a width allowing that at the production, each layer can be secured individually to the adhesive strip in a way that the layers are slightly overlapped one above the other, while the outermost part of the covering film provided with the adhesive remains exposed for the purpose of securing it to an intact skin surface. After the mat has been placed on a body surface to be treated, the periphery of the covering film provided with the adhesive adheres to the skin and in this way the covering film presses the layers of the mat to the body surface so that they cannot substantially move relative to each other.

In an alternative way of securing the layers to each other, the layers are weakly compressed at production, or the separate layers are individually placed on each other before use, followed by securing the multilayer mat to the body surface by means of a covering film with an adhesive edge.

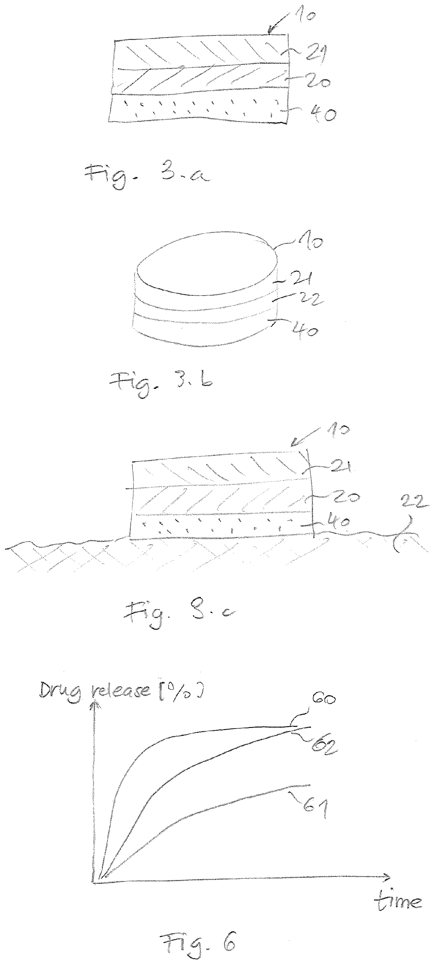

A preferred embodiment of the multilayer mat according to the present invention is illustrated in Figures 3a-c. As shown in Figures 3a-c, this embodiment of the multilayer mat 10 comprises an additional membrane layer 40 on the outer side of the membrane layer 20. In this embodiment, when the multilayer mat 10is secured to the skin surface 22, the membrane layer 40 contacts with the body surface 22. The membrane layer 40 is preferably a microfibrous polymer layer, in which the thickness of the polymer fibres is preferably at most 10 µm, more preferably 1-10 µm. The function of the membrane layer 40 is to increase the mechanical strength of the multilayer mat 10. When using a microfibrous membrane layer 40, which contacts the skin, this layer tends to slightly swell due to the moisture produced by the body surface, while permeating the moisture to the following water-insoluble nanofibrous membrane layer 20. During this process, however, the microfibrous membrane layer 40 is keeping its fibrous structure permanently. The material of the microfibrous membrane layer 40 may be a polymer that is either non-degradable or degradable under physiological conditions. The use of this additional layer is particularly useful in those systems (primarily in medical applications), wherein the absorption of the mat is expected.

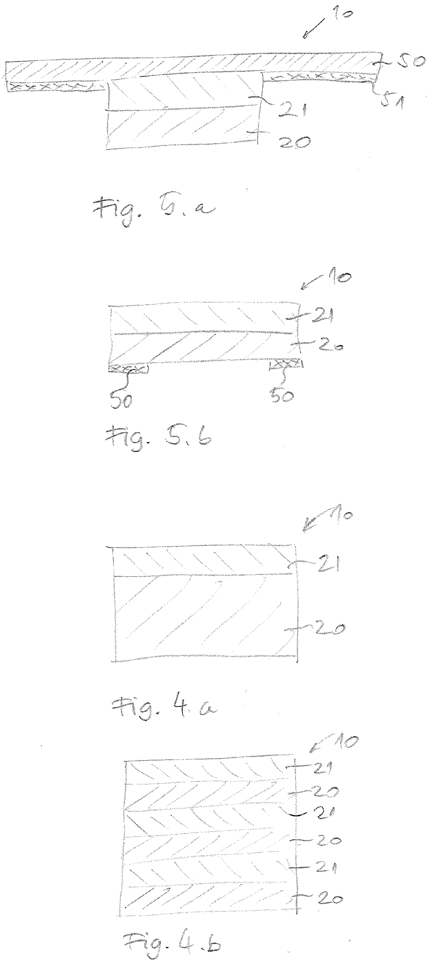

Further two preferred embodiments of the multilayer mat 10 according to the present invention are shown in Figures 4a and 4b in schematic lateral sectional views. The embodiment of the multilayer mat 10 depicted in Figure 4a comprises a membrane layer 20 having an increased thickness, which results in that the humour produced by the skin gets more slowly into the carrier layer 21, and from the carrier layer 21 the solution containing the active agent also returns more slowly to the body surface, thereby the release process of the active agent slows down and the release period prolongs. With this embodiment, it should be taken into account that during the treatment a major portion of the solution containing the active agent is staying in the thicker membrane layer 20 and a corresponding amount of the active agent remains eventually within the mat, except the case when a mat made of an absorbable polymer is used and the complete absorption of the mat is bode.

The multilayer mat 10 according to the present invention may comprise even a plurality of carrier layers and a plurality of membrane layers alternately. In the embodiment shown in Figure 4b, the multilayer mat 10 comprises three membrane layers 20 and three carrier layers 21, which are arranged alternately. The first membrane layer 20 is in direct contact with the skin surface. In this case the release mechanism of the active agent differs from the release mechanism of the two-layer structure shown in Figure 2 only in that the humour absorbed from the skin reaches not only the carrier layer nearest the skin, but through this layer it partly diffuses further into to the following membrane layers. From these membrane layers the humour gets into further carrier layers and in those layers it also produces its dissolving effect. Then the active agent releasing from the farther carrier layers starts to flow by diffusion, in the form of a solution towards the skin , through via the underneath membrane layers and carrier layers (which are closer to the skin).

The advantage of the resulting multilayer structure is that the release of the active agent is slower and the same concentration level can be provided from the mat for a longer period as compared to the two-layer structure shown in Figures 1a-c. However, essentially the whole amount of the active agent can be released from the mat and a specific dosing rate (i.e. dose weight per time unit) can be maintained for a longer time (which, of course, involves some surplus of the active agent) or at least the amount of the residual active agent is considerably less as compared to the mat comprising a thickened membrane layer shown in Figure 4a. In the multilayer mat 10, the release time of the active agent can be prolonged by the multiplication of the different layers.

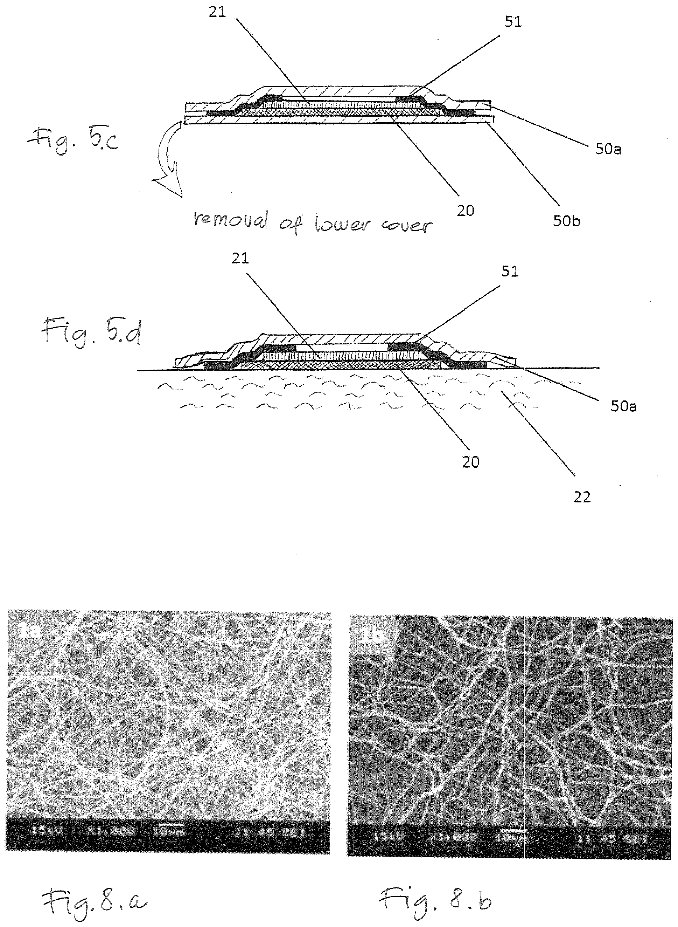

Figures 5a-d show further embodiments of the multilayer mat according to the present invention, said embodiments allowing their securing to the body surface.

As shown in Figure 5a, the multilayer mat 10 according to the present invention is comprises a fastening member 50 for securing the mat to the body surface 22. A free part of one side of the fastening member 50 (facing to the body surface 22 when used) comprises an adhesive layer 51 to be stuck to the skin. The fastening member 50 is secured to the unit comprising the at least one membrane layer 20 and the at least one carrier layer 21 in such a way that the fastening member 50 at least partially extends beyond the contour of said unit. The fastening member 50 can be stuck to the body surface 22 along its surface having the adhesive layer 51, thereby the multilayer mat 10 can be secured to the skin surface to be treated.

Figure 5b illustrates an embodiment of the multilayer mat 10 according to the present invention, wherein the fastening member 50 itself is formed as an adhesive layer that is provided on the outer side of the terminal membrane layer 20, along the periphery thereof.

The embodiment shown in Figures 5c-d differs from the embodiment shown in Figure 5a in that one side of an upper covering film 50a is coated with an adhesive layer 51, and the carrier layer 21 and the membrane layer 20 are both adhered thereto in such a way that the membrane layer 20 extends beyond the carrier layer 21. Preferably, this embodiment also comprises an further covering film 50b on the opposite side of the mat, wherein the covering film 50b covers the membrane layer 21 completely and is secured to the covering film 50a by the adhesive layer 51. Before use the covering film 50b should be removed from the mat and the mat should be stuck to the body surface 22 to be treated by using the covering film 50a.

Figure 6 illustrates the profile of the release of the active agent in different embodiments of the multilayer mat according to the present invention as a time function of the released amount of the active agent. The horizontal axis shows the time measured from the beginning of the treatment and the vertical axis shows the percentage ratio of the active agent released from the total amount of the active agent as a function of time. The curve 60 shown in the diagram refers to a two-layer mat which comprises a membrane layer of unit thickness and a carrier layer of unit thickness, the curve 61 refers to a two-layer mat which comprises a membrane layer of three-unit thickness and a carrier layer of unit thickness, and the curve 62 refers to a six-layer mat in which the membrane layers of unit thickness and the carrier layers of unit thickness are repeated alternately. In the diagram the qualitative differences between the release profiles of the active agent at the different mat structures can be clearly seen, which have already been mentioned above with respect to the various embodiments. This means that by increasing the thickness of the membrane layer or by arranging the layers in an alternating pattern, the release of the active agent can be slowed down according to different unique profiles. It can be established on the basis of the completed tests that the alternating structure allows more dynamic release of the active agent in a cascaded fashion, whereas a structure comprising two layers with a thickened membrane layer results in more passive release of the active agent, wherein due to the increased thickness of the membrane layer there is some delay in the release of the active agent and the time needed for the active agent to reach the body surface is also somewhat longer.

Figures 7a-b show the fibre structure of the nanofibrous layers of the multilayer mat according to the present invention in electron microscope images. The electron microscope image of the water-soluble nanofibrous carrier layer is shown in Figure 7a and the electron microscope image of the water-insoluble nanofibrous membrane layer (which cannot be dissolved in water at all or can be dissolved in water only to a very limited extent) is shown in Figure 7b. The PVA nanofibrous structure (membrane layer) shown in Figure 7b is produced by a heat treatment of the PVA nanofibrous structure (carrier layer) shown in Figure 7a, as the water-soluble PVA nanofibrous structure renders into a water-insoluble structure due to the heat treatment.

This effect is of importance concerning the production of the multilayer mat according to the present invention, since it is enough to produce only one kind of water-soluble nanofibrous plates from a mono- or multi-component polymer material, which becomes water-soluble after heat treatment (or through another treatment). Hence, after the heat treatment the same material can be used as a water-insoluble nanofibrous structure during the production of the mat.

For the material of the membrane layer, not only those polymers, which can be rendered to water-insoluble through a heat treatment, can be used, but also polymers, which can rendered water-insoluble through any other treatments, or even primarily water-insoluble polymers may also be used.

The multilayer mat according to the present invention is preferably made in the form of a plate, however, on demand it can be produced as rolls. In this latter case, the plates ready for treatment should be cut from the roll one by one, in the desired size.

In the following we will describe various possible applications of the multilayer mat according to the present invention.

The multilayer mat can be used for the local application of an active agent that can absorb through any body surface (preferably on an injured body surface) producing sanies or any other humour during a treatment, or through moistened intact skin surface. The solution according to the present invention is particularly suitable for medical purposes, in particular for the local application of analgesics, tissue regeneration improvers, haemostatics, antibiotics, local anaesthetics through an injured and humour-producing body surface. Another advantage of the present invention is that by the local use of the highly toxic antibiotics, the dose required for the treatment can be considerably decreased with the use of the mat according to the present invention. Thereby the systemic loading can be significantly decreased, while a long-term use can be provided on the surface to be treated.

The multilayer mat can be applied mainly on open wounds, so it could help, for example, in the formation of skin islands in burns thus eliminating even the need of epidermis implantation, as it is well known that the microfibres and the nanofibres are capable of stimulating cell proliferation.

The multilayer mat according to the present invention can be used for cosmetics purposes too, wherein the active agent is not necessarily a medical composition, but it may be an agent producing a skin conditioning, regenerating, refreshing etc. effect (for example, vitamins to be applied through the skin). In this case, as mentioned above, it is recommended that before the application of the mat the treated skin surface should be moistened in order to activate the active agent.

A further benefit of the multilayer mat is that the dose may be reduced even due to the fact that some poorly soluble crystalline active agents can be reserved in a stable amorphous form in the nanofibrous polymer system. The amorphous form may result in higher dissolubility, a reduced dose and higher bioavailability.

When the multilayer mat is used on a wound surface, in addition to the local application of the active agent, the polymer microfibrous layer also stimulates the proliferation of the fibroblasts.

The nanofibrous and/or microfibrous membrane layer of the multilayer mat may be made from a biodegradable polymer too. Due to the use of a biodegradable polymer, the mat should not be changed, because it degrades in course of time, and after degradation it even absorbs through the injured, humour-producing body surface in case of specific polymers.

## Claims

1. A multilayer mat (10) for dispensing an active agent through a body surface, said mat comprising at least one membrane layer (20) and at least one carrier layer (21) containing the active agent, **characterized in that**
- the at least one carrier layer (21) is formed by a solid nanofibrous layer made of at least one biocompatible, water-soluble polymer, said carrier layer containing the active agent in a solid state,
- the at least one membrane layer (20) is formed by a solid nanofibrous layer made of at least one biocompatible, water-insoluble polymer, said membrane layer being permeable for both moisture and a solution of the active agent,
- wherein each of the at least one carrier layer (21) and the at least one membrane layer (20) has a predefined thickness.

2. The multilayer mat according to claim 1, comprising a plurality of carrier layers (21) and a plurality of membrane layers (20) that are arranged alternately.

3. The multilayer mat according to claim 1, comprising a single carrier layer (21) and a single membrane layer (20), wherein the thickness of the membrane layer (20) is defined as a function of a predetermined release rate of the active agent.

4. The multilayer mat according to any one of claims 1 to 3, wherein the thickness of the membrane layer (20) is about 0.05-1 mm, preferably about 0.05-0.5 mm.

5. The multilayer mat according to any one of claims 1 to 4, wherein further comprising an additional micro fibrous membrane layer (40) on the outer side of the terminal membrane layer (20).

6. The multilayer mat according to any one of claims 1 to 5, further comprising a fastening member (50) for securing to the body skin.

7. The multilayer mat according to claims 6, wherein the fastening member (50) is formed by a covering layer arranged on the outer side of a terminal nanofibrous or microfibrous membrane layer (20, 40), said covering layer extending beyond the contour of the unit of the at least one membrane layer (20) and the at least one carrier layer (21), and provided with an adhesive (51) on the free surface thereof facing toward said layers.

8. The multilayer mat according to claims 6, wherein the fastening member (50) is formed by an adhesive layer applied on the outer side of a terminal nanofibrous or microfibrous membrane layer (20, 40), along the periphery thereof.

9. The multilayer mat according to any one of claims 1 to 8, wherein the membrane layer (20, 40) and/or the carrier layer (21) is made of a polymer, which is degradable under physiological conditions, in particular a polymer, which can be degraded in an enzymatic or other biological way.
